# EUROPÄISCHE PATENTSCHRIFT

(11) **EP 1 733 215 B1**
(45) Veröffentlichungstag und Bekanntmachung des Hinweises auf die Patenterteilung: **27.05.2009**
(21) Anmeldenummer: 05716474.1
(22) Anmeldetag: 31.03.2005
(51) Int. Cl.: G01N 27/407

(54) **GASSONDE MIT HYGROSKOPISCH BESCHICHTETER SCHUTZEINRICHTUNG**
GAS PROBE WITH HYGROSCOPIC COATED PROTECTION DEVICE
SONDE DE GAZ POURVUE D'UN DISPOSITIF DE PROTECTION A REVETEMENT HYGROSCOPIQUE

(30) Priorität: 07.04.2004 DE 102004017586
(43) Veröffentlichungstag der Anmeldung: 20.12.2006
(73) Patentinhaber: Emitec Gesellschaft für Emissionstechnologie mbH, 53797 Lohmar (DE); AUDI AKTIENGESELLSCHAFT, 85045 Ingolstadt (DE)
(72) Erfinder: BRÜCK, Rolf, 51429 Bergisch Gladbach (DE); PFALZGRAF, Bernhard, 85051 Ingolstadt (DE); ODENDALL, Bodo, 86633 Neuburg (DE)
(74) Vertreter: Rössler, Matthias
(86) Internationale Anmeldenummer: PCT/EP2005/003374
(87) Internationale Veröffentlichungsnummer: WO 2005/098410

(56) Entgegenhaltungen:
- DE-A1- 3 818 736
- DE-A1- 10 159 858
- US-A- 5 762 771

## Beschreibung

Die Erfindung betrifft eine Gassonde, insbesondere eine Lambda-Sonde zur Analyse von Abgasen einer mobilen Verbrennungskraftmaschine.

Solche Gassonden weisen üblicherweise ein sensitives Bauteil (Sensorelement) auf, welches zur Analyse von Gasen, insbesondere Abgasen in Abgassystemen von Verbrennungsmotoren, eingesetzt werden können. Das Sensorelement ist zum Schutz vor Beschädigungen infolge mechanischer und thermischer Einwirkung üblicherweise von einem Schutzrohr umgeben, das durch geeignete Öffnungen den Zutritt des zu analysierenden Gases zu dem innerhalb des Schutzrohres angeordneten Sensorelement der Lambda-Sonde erlaubt. Dieses Schutzrohr dient in erster Linie der Vermeidung von Transportschäden und Einbauschäden, sowie der Vermeidung einer Thermoschockbelastung des beheizten sensitiven Bauteils der Lambda-Sonde durch Kontakt mit Wassertropfen, die sich aus Wasserdampf des Gasstromes bilden.

So ist beispielsweise aus der US 5,762,771 ein Sensor zur Erfassung eines Luft-Treibstoff-Gemischverhältnisses bekannt, der in einer Ausführungsform mit einer Schutzeinrichtung versehen ist, die ein hygroskopisches Material enthält.

Ferner ist aus der DE 101 59 858 A1 ein Gasmessgliedelement und ein Gassensor bekannt. Dabei ist eine Messgaselektrode auf einer Oberfläche eines festen elektrolytischen Substrats derart bereitgestellt, dass sie einem zu messenden Gas ausgesetzt ist. Der Grundkörper des Sensors ist mit einer hygroskopischen Beschichtung versehen.

Aufgabe der vorliegenden Erfindung ist es, eine Gassonde zu schaffen, welche eine erhöhte Sicherheit gegenüber bekannten Gassonden dagegen aufweist, dass das Sensorelement mit Wassertopfen in Kontakt kommt. Darüber hinaus soll die Gassonde einfach aufgebaut und im Rahmen einer Serienfertigung ohne großen Aufwand und kosteneffektiv herstellbar sein. Auch sollen die bekannten technischen Probleme mit den derzeit verfügbaren Gassonden zumindest abgeschwächt werden.

Diese Aufgaben werden gelöst mit einer Gassonde gemäß den Merkmalen des unabhängig formulierten Vorrichtungsanspruchs sowie einem Verfahren zur Herstellung einer Gassonde gemäß dem unabhängig formulierten Verfahrensanspruch. Weitere vorteilhafte Ausgestaltungen sind in den jeweils abhängig formulierten Patentansprüchen beschrieben. Es ist darauf hinzuweisen, dass jede, technologisch sinnvolle, Kombination der in den Patentansprüchen genannten Merkmale, gegebenenfalls unter Einbeziehung von Merkmalen der Beschreibung, zu weiteren, vorteilhaften Ausgestaltungen der Erfindung führen kann.

Die erfindungsgemäße Gassonde, insbesondere eine Lambda-Sonde zur Analyse von Abgasen einer mobilen Verbrennungskraftmaschine, hat mindestens eine Schutzeinrichtung, die ein sensitives, mit einem Gas in Kontakt kommendes Bauteil (Sensorelement) der Gassonde zumindest bereichsweise umgibt. Die Gassonde ist dadurch gekennzeichnet, dass die mindestens eine Schutzeinrichtung eine Gaskontaktfläche hat, die zumindest teilweise eine hygroskopische Oberfläche aufweist. Dabei ist die hygroskopische Oberfläche wenigstens teilweise mit einer Beschichtung gebildet, die vorteilhafterweise ein hochtemperaturfestes Trockenmittel umfasst. Als Trockenmittel können beispielsweise verschiedene Salze oder auch organische Substanzen eingesetzt werden. Die Beschichtung ist vorteilhafterweise auch wasserschlagfest sowie insbesondere widerstandsfähig gegen Erosion und Korrosion.

Die mindestens eine Schutzeinrichtung, unter Umständen können hier zwei oder drei solcher Schutzeinrichtungen (gegebenenfalls konzentrisch zueinander überdeckend angeordnet) vorgesehen sein, stellt einerseits einen Schutz gegen Transport- und/oder Einbauschäden dar, ist also relativ stabil ausgeführt. Weiterhin trägt die Schutzeinrichtung dafür Sorge, dass das Sensorelement nicht mit im Gasstrom enthaltenen Verunreinigungen (Partikel, Ruß, etc) und/oder Wassertropfen in Kontakt kommt. Gleichzeitig ermöglicht die Schutzeinrichtung jedoch den Zugang des zu analysierenden Gases hin zum Sensorelement. Üblicherweise stellt eine solche Schutzeinrichtung eine Art Gehäuse, Kappe, Rohr, Gitter, etc. dar, wobei Öffnungen vorgesehen sind, durch die das Gas hinein zu innen liegenden Bereichen mit dem Sensorelement strömen kann.

Es wird nun vorgeschlagen, dass die Schutzeinrichtung, insbesondere alle Schutzeinrichtungen, zumindest teilweise an den Flächen, die mit dem zu analysierenden Gasstrom in Kontakt kommen, eine hygroskopische Oberfläche aufweist. Mit "hygroskopisch" ist insbesondere gemeint, dass die Oberfläche in der Lage ist, Wasserdampf aus dem Gasstrom aufzunehmen bzw. die Bildung von Wassertropfen auf der Gaskontaktfläche zu verhindern. Zu diesem Zweck kann die Gaskontaktfläche selbst hygroskopisch sein, beispielsweise durch besondere Ausgestaltung der Oberflächenrauhigkeit und/oder der Porosität. Es ist jedoch auch möglich, dass die Gaskontaktfläche mit einer zusätzlichen Schicht aus einem anderen, von dem Material der Schutzeinrichtung verschiedenen, Material bedeckt ist.

Weiter wird vorgeschlagen, dass die Gassonde eine Beschichtung mit einer Schichtdicke im Bereich von 10 µm (Mikrometer) bis 50 µm hat. Bevorzugt beträgt die Schichtdicke 15 µm bis 25 µm. Aufgrund der starken thermischen Belastung beispielsweise im Abgassystem eines Automobils ist eine besondere Haftfestigkeit der Beschichtung auf der Schutzeinrichtung erforderlich. Um einen dauerhaften Verbleib der Beschichtung auf der Schutzeinrichtung zu gewährleisten, ist die Schichtdicke relativ klein ausgeführt, so dass ein Abplatzen vermieden wird.

Gemäß einer weiteren Ausgestaltung der Gassonde weist die Beschichtung eine Porosität auf, so dass das Porenvolumen pro Volumeneinheit der Beschichtung in einem Bereich von 30 % bis 90 % liegt. Das heißt mit anderen Worten, dass bezogen auf eine vorgegebene Volumeneinheit das von den Poren gebildete Volumen (statistisch gemittelt) 30 % bis 90 %, bevorzugt um 50 %, ausmacht.

Es wird auch vorgeschlagen, dass die Gassonde eine Oxid-Beschichtung umfasst, insbesondere das die Beschichtung zumindest ein Oxid aus Titanoxid, Zirkonoxid und Aluminiumoxid umfasst. Diese hochtemperaturfesten Oxide begünstigen die hygroskopische Wirkung der Beschichtung.

Gerade bei der Anordnung einer solchen Gassonde im Abgassystem eines Automobils ist es vorteilhaft, wenn die Beschichtung katalytisch wirkende Bestandteile aufweist, insbesondere ein Edelmetall (z.B. Platin, Rhodium,...). Das bedeutet, dass die mit dem Abgas in Kontakt befindliche Oberfläche gleichzeitig zur Umsetzung von im Abgas enthaltenen Schadstoffen verwendet wird. Um jedoch die Zusammensetzung des zu analysierenden Gasstromes nicht in relevantem Maße zu beeinträchtigen, wird generell auch vorgeschlagen, dass die Beschichtung keine Wirkung (z.B. Speicherfähigkeit) auf die Bestandteile des Gasstromes hat, die von dem Gassonde messtechnisch bestimmt werden, beispielsweise Sauerstoff.

Gemäß einer Weiterbildung der Gassonde weisen auch die mit dem Gas in Kontakt kommenden weiteren Komponenten wenigstens teilweise eine hygroskopische Oberfläche auf, insbesondere ein Grundkörper der Gassonde. Der Grundkörper stellt die Aufnahme des sensitiven Bauteils dar und hat üblicherweise am äußeren Umfang ein Gewinde, um die Gassonde in einem Gehäuse zu fixieren. Um zu verhindern, dass sich an diesem Grundkörper Wassertropfen bilden, ist auch dieser mit einer hygroskopischen Oberfläche ausgestattet. Alternativ oder in Kombination dazu kann diese hygroskopische Oberfläche mittels wenigstens einem separaten Formköpers gebildet sein, der die betreffende Komponente zumindest teilweise abdeekt bzw. umschließt. Dieser Formkörper kann stoffschlüssig und/oder formschlüssig, insbesondere unverlierbar, zu der Komponente bzw. mehreren Komponenten ausgeführt sein.

Unter Berücksichtigung der kostengünstigen Herstellung einer solchen Gassonde wird weiter vorgeschlagen, dass die mindestens eine Schutzeinrichtung eine Kappe ist, die ein hochtemperaturfestes, tiefziehbares Material umfasst. Die Kappe kann so gerade für die Serienfertigung in hoher Stückzahl sehr präzise hergestellt werden. Als Kennzeichen für ein tiefziehbares Material wird hier insbesondere gefordert, dass dieses ein Grenzziehverhältnis (βₘₐₓ) im Bereich von 1,6 bis 2,0 hat. Das Grenzziehverhältnis wird mittels einer Tiefziehprüfung (z.B. nach Swift) ermittelt, wobei aus Blechronden mit stufenweise vergrößertem Durchmesser (d₀ₘₐₓ) bei gleich bleibendem Stempeldurchmesser (d₁) zylindrische Näpfe gezogen werden. Kennwert ist das Grenzziehverhältnis, bei dem die Grenze der Ziehfähigkeit des Bleches durch einen gerade noch nicht eintretenden Bodenriss erreicht ist (βₘₐₓ= d₀ₘₐₓ / d₁).

Die Gassonde, die mindestens eine Schutzeinrichtung und einen Grundkörper umfasst, stellt mindestens eine Oxid-bildende Oberfläche der Komponenten bereit. Das heißt, dass die Schutzeinrichtung und/oder der Grundkörper zumindest teilweise so thermisch behandelt werden kann, dass sich auf ihrer Oberfläche ein Oxid bildet, insbesondere Aluminiumoxid. Dieses Oxid ermöglicht, dass die nachfolgende Beschichtung sich dauerhaft auch unter hohen thermischen und dynamischen Belastungen auf der Oberfläche applizieren lässt.

Vorteilhaft ist auch die Ausgestaltung einer Gassonde, wobei die mindestens eine Schutzeinrichtung elektrisch beheizbar ist. Dies kann insbesondere kurzzeitig und diskontinuierlich erfolgen, um das in der Beschichtung festgehaltene Wasser zu verdampfen. Dabei ist es vorteilhaft, dies in Situationen durchzuführen, in denen das Abgas eine relativ niedrige Temperatur hat, beispielsweise vor oder unmittelbar nach dem Motorstart bzw. in Leerlaufphasen des Motors.

Gemäß einem weiteren Aspekt der vorliegenden Erfindung wird ein Verfahren zur Herstellung einer Gassonde, insbesondere einer Lambda-Sonde zur Analyse von Abgasen einer mobilen Verbrennungskraftmaschine vorgeschlagen. Die Gassonde hat mindestens eine Schutzeinrichtung, die ein sensitives, mit einem Gas in Kontakt kommendes Bauteil (Sensorelement) der Gassonde zumindest bereichsweise umgibt, sowie einen Grundkörper. Es wird nun vorgeschlagen, dass die mindestens eine Schutzeinrichtung oder der Grundkörper zumindest teilweise mit einer hygroskopischen Beschichtung versehen wird. Auf diese Weise lässt sich insbesondere ein erfindungsgemäßes Gassonde herstellen, wie es vorstehend beschrieben ist.

Dabei sind ggf. freie Bereiche vorzusehen, insbesondere dort, wo die Oberfläche nahe anderen Funktionsflächen angeordnet ist. Funktionsflächen haben eine vordergründige Funktion, zum Beispiel dienen sie als Verbindungsflächen von Komponenten miteinander, als (elektrische) Kontaktflächen, als Kennzeichnung, etc.. So ist es beispielsweise vorteilhaft, dass der Grundkörper und/oder die Schutzeinrichtung an den Stellen keine Beschichtung aufweist, wo eine formschlüssige (Quetsch-)Verbindung und/oder eine fügetechnische (Schweiß-, Löt-, o.ä.)Verbindung miteinander bzw. mit weiteren Komponenten des Gassensors vorgesehen sind.

Dabei ist es besonders vorteilhaft, dass die mit der Beschichtung zu versehenden Oberflächen vorbehandelt werden, insbesondere auf den Oberfläche ein Oxid gebildet wird. Die Bildung eines Oxids auf der Oberfläche kann durch eine thermische Behandlung erfolgen, wenn entsprechende Bestandteile des Materials der mindestens einen Schutzeinrichtung bzw. des Grundkörpers vorliegen. Es ist aber auch möglich, dass Material in einem zusätzlichen Fertigungsschritt zunächst auf der Oberfläche zu applizieren und anschließend ein Auflegieren des Materials zu bewirken.

Es ist unter Umständen vorteilhaft; dass die mindestens eine Schutzeinrichtung oder der Grundkörper vor dem Beschichten aluminiert werden. Das bedeutet, dass beispielsweise eine Aluminium-Knetlegierung auf der Oberfläche appliziert und durch eine anschließende thermische Behandlung auf der Oberfläche fixiert wird. Die Bereitstellung von Aluminium ermöglicht die nachträgliche Ausbildung von Aluminiumoxid, welches als Trägerschicht für die nachfolgende hygroskopische Beschichtung besonders gut geeignet ist.

In diesem Zusammenhang ist es besonders vorteilhaft, dass die mindestens eine Schutzeinrichtung ein umgeformtes, insbesondere tiefgezogenes, Teil ist, bei dem der Aluminier-Vorgang vor dem Umformen stattfindet und vorteilhafterweise nach dem Umformschritt ein Diffusionsglühen durchgeführt wird.

Gemäß einer weiteren vorteilhaften Ausgestaltung des Verfahrens umfasst das Auftragen der Beschichtung zumindest einen der folgenden Fertigungsschritte: Spritzen, Tauchen, Sprühen, Thermisches Spritzen.

Beim Spritzen bzw. Sprühen wird die Beschichtung unter Einsatz eines Trägermediums, beispielsweise Luft, auf die zu beschichtende Oberfläche appliziert. Beim Tauchen wird das zu beschichtende Teil in ein Bad aus der Beschichtung eingetaucht. Zur Bereitstellung einer dauerhaften. Beschichtung sind bei den drei vorstehend genannten Verfahren die Ausbildung einer Oxidbeschichtung zuvor vorteilhaft. Auf eine solche Oxid-bildende Maßnahme kann beim Applizieren der Beschichtung mittels des Thermischen Spritzens verzichtet werden, da die hierbei eingesetzten Temperaturbereiche einen ausreichenden Stoffschluss bzw. ausreichende Adhäsionskräfte zwischen der Oberfläche und der Beschichtung gewährleisten. Beim Thermischen Spritzen werden üblicherweise Temperaturen beim Applizieren von über 500 °C eingesetzt, bevorzugt oberhalb von 900° (Plasma-Spritzen), wobei ggf. kleinere Schichtdicken (z.B. kleiner 40 µm) vorgesehen werden können.

Zur Gewährleistung einer gleichmäßigen Verteilung der Beschichtung bzw. einer überwiegend gleichen Schichtdicke können weitere Bearbeitungsschritte erforderlich sein, beispielsweise ein Rotieren des Teils (Ausnutzung von Zentrifugalkräften)), ein Abklopfen (Ausnutzung einer Impulswirkung), ein Ausblasen (Einsatz eines unter Überdruck stehenden Gasstromes), etc.. Insbesondere ist zu gewährleisten, dass die Gaseintrittsöffnungen bzw. Gasaustrittsöffnungen der Schutzeinrichtung nicht über die Maßen verschlossen sind.

Besonders vorteilhaft ist die Gassonde in Kombination mit einer Abgasreinigungskomponente. Der Begriff "Abgasreinigungskomponente" soll als Oberbegriff für alle zur Abgasbehandlung geeigneten Komponenten stehen, insbesondere für katalytische Konverter, Strömungsmischer, Filter, Adsorber, Kohlenwasserstoff- oder Rußfallen.

Besonders vorteilhaft ist die Bereitstellung einer Gassonde im Abgassystem eines Fahrzeuges, insbesondere eines Automobils.

Die Erfindung sowie das technische Umfeld wird nachfolgend anhand der Figuren näher erläutert. Dabei ist darauf hinzuweisen, dass besonders bevorzugte Ausführungsbeispiele der Erfindung dargestellt sind, auf die die Erfindung jedoch nicht begrenzt ist. Es zeigen:
- Fig. 1: schematisch eine Detailansicht einer Gassonde, und
- Fig. 2: einen Querschnitt durch die Gassonde gemäß Fig. 1.

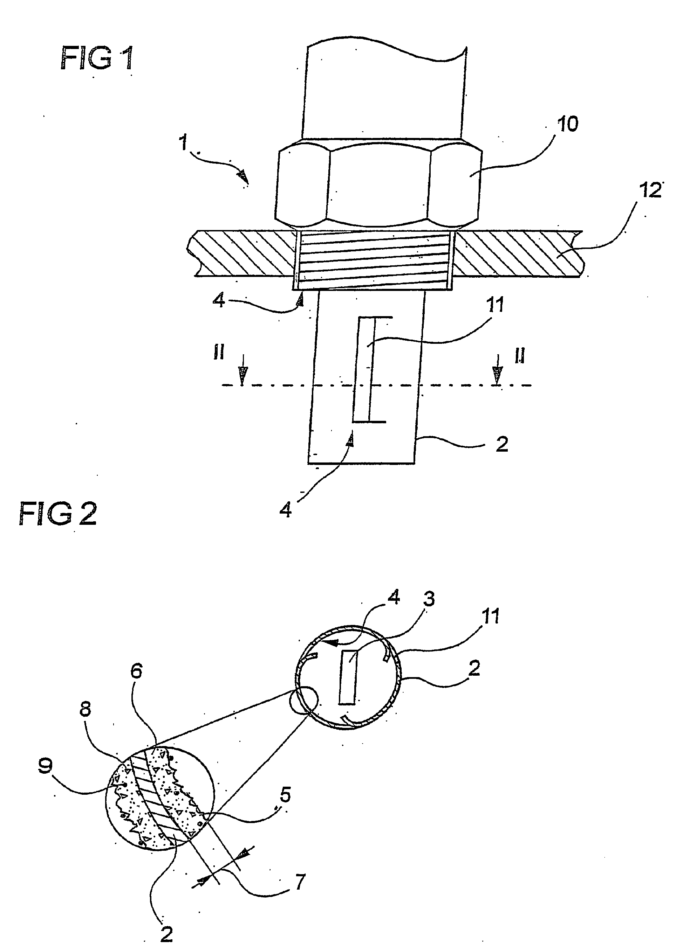

Fig. 1 zeigt schematisch den Aufbau einer Gassonde 1 mit einem Grundkörper 10 und einer Schutzeinrichtung 2. Die Gassonde 1 ist durch ein Gehäuse 12 (beispielsweise das Gehäuse einer Abgasleitung oder einer Abgasreinigungskomponente) hindurch gesteckt und mit diesem befestigt. Dabei ist ein Teil der Gassonde 1 umfassend die Schutzeinrichtung 2 mit dem zu analysierenden Gasstrom in Kontakt. Sowohl die Schutzeinrichtung 2 als auch der Grundkörper 10 weist eine oder mehrere Gaskontaktflächen 4 auf, also Oberflächen, die mit dem zu analysierenden Gas in Kontakt treten. Dabei weist der zu analysierende Gasstrom zumindest zeitweise Wasserdampf auf, der unter Umständen an den Gaskontaktflächen 4 kondensieren kann. Im Inneren der Schutzeinrichtung 2 ist das Sensorelement 3 angeordnet (Fig. 1 nicht dargestellt). Der Kontakt des zu analysierenden Gasstromes mit dem sensitiven Bauteil (Sensorelement 3) wird über Öffnungen 11 in der Schutzeinrichtung 2 ermöglicht.

Wie dies in Fig. 1 angedeutet ist, zeigt Fig. 2 einen Querschnitt durch die Schutzeinrichtung 2 der Gassonde 1. Die Schutzeinrichtung 2 ist hier als im wesentlichen zylinderförmiges Teil dargestellt, welches auch auf seiner Stirnseite verschlossen ist. Am Umfang der Schutzeinrichtung 2 sind mehrere Öffnungen 11 als Schlitze vorgesehen, die ein Gasaustausch mit den innenliegenden Bereichen ermöglichen. Die Schutzeinrichtung 2 weist innen und außen eine Beschichtung 5 auf, die in dem vergrößerten Teilbereich links unten in der Fig. 2 schematisch dargestellt ist.

Die Beschichtung 5 ist auf den innenliegenden und außenliegenden Gaskontaktflächen 4 der Schutzeinrichtung 2 vorgesehen. Die Beschichtung 5 hat eine Schichtdicke 7 im Bereich von 10 µm bis 50 µm. Der Anteil der Poren 8 der Beschichtung 5 pro Einheitsvolumen der Beschichtung 5 beträgt etwa 50 %. Die Beschichtung 5 weist Trockenmittel 6 zur Aufnahme von Wasserdampf aus dem Gasstrom sowie Edelmetalle 9 zur katalytischen Umsetzung von Schadstoffen im Abgas auf.

Die hier vorgeschlagene Gassonde erhöht die Betriebssicherheit gerade in Abgasanlagen von Automobilen erheblich. Zudem lässt sich die Gassonde einfach auch im Rahmen einer Serienfertigung herstellen.

### Bezugszeichenliste

- 1: Gassonde
- 2: Schutzeinrichtung
- 3: Bauteil
- 4: Gaskontaktfläche
- 5: Beschichtung
- 6: Trockenmittel
- 7: Schichtdicke
- 8: Pore
- 9: Edelmetall
- 10: Grundkörper
- 11: Öffnung
- 12: Gehäuse

## Patentansprüche

1. Gassonde (1), insbesondere Lambda-Sonde zur Analyse von Abgasen einer mobilen Verbrennungskraftmaschine, mit mindestens einer Schutzeinrichtung (2), die ein sensitives, mit einem Gas in Kontakt kommendes, Sensorelement (3) der Gassonde (1) zumindest bereichsweise umgibt, **dadurch gekennzeichnet, dass** die mindestens eine Schutzeinrichtung (2) eine Gaskontaktfläche (4) hat, die zumindest teilweise eine hygroskopische Oberfläche aufweist, wobei die hygroskopische Oberfläche wenigstens teilweise mit einer Beschichtung (5) gebildet ist, die vorteilhafterweise ein hochtemperaturfestes Trockenmittel (6) umfasst.

2. Gassonde (1) nach Anspruch 1, **dadurch gekennzeichnet, dass** die Beschichtung (5) eine Schichtdicke (7) im Bereich von 10 µm bis 50 µm hat.

3. Gassonde (1) nach Anspruch 1 oder 2, **dadurch gekennzeichnet, dass** die Beschichtung (5) eine Porosität aufweist, so dass das Porenvolumen pro Volumeneinheit der Beschichtung (5) in einem Bereich von 30% bis 90% beträgt.

4. Gassonde (1) nach einem der Ansprüche 1 bis 3, **dadurch gekennzeichnet, dass** die Beschichtung (5) ein Oxid umfasst, insbesondere zumindest eines aus Titanoxid, Zirkonoxid und Aluminiumoxid.

5. Gassonde (1) nach einem der Ansprüche 1 bis 4, **dadurch gekennzeichnet, dass** die Beschichtung (5) katalytisch wirkende Bestandteile aufweist, insbesondere ein Edelmetall (9).

6. Gassonde (1) nach einem der Ansprüche 1 bis 5, **dadurch gekennzeichnet, dass** auch die mit dem Gas in Kontakt kommenden weiteren Komponenten wenigstens teilweise eine hygroskopische Oberfläche aufweisen, insbesondere ein Grundkörper (10) der Gassonde (1).

7. Gassonde (1) nach einem der Ansprüche 1 bis 6, **dadurch gekennzeichnet, dass** die mindestens eine Schutzeinrichtung (2) eine Kappe ist, die ein hochtemperaturfestes, tiefziehbares Material umfasst.

8. Gassonde (1) nach einem der Ansprüche 1 bis 7, wobei die Gassonde (1) zumindest folgende Komponenten umfasst: mindestens eine Schutzeinrichtung (2) und einen Grundkörper (10); **dadurch gekennzeichnet, dass** wenigstens eine Komponente aus einem Material ist, welches eine Oxid-bildende Oberfläche bereitstellt.

9. Gassonde (1) nach einem der Ansprüche 1 bis 8, **dadurch gekennzeichnet, dass** die mindestens eine Schutzeinrichtung (2) elektrisch beheizbar ist.

10. Verfahren zur Herstellung einer Gassonde (1) nach einem der vorhergehenden Ansprüche, insbesondere einer Lambda-Sonde zur Analyse von Abgasen einer mobilen Verbrennungskraftmaschine, mit mindestens einer Schutzeinrichtung (2), die ein sensitives, mit einem Gas in Kontakt kommendes Sensorelement (3) der Gassonde (1) zumindest bereichsweise umgibt, sowie einem Grundkörper (10), bei dem wenigstens die mindestens eine Schutzeinrichtung (2) zumindest teilweise mit einer hygroskopischen Beschichtung (5) versehen wird.

11. Verfahren nach Anspruch 10, bei dem die mit der Beschichtung (5) zu versehenden Oberflächen vorbehandelt werden, insbesondere ein Oxid gebildet wird.

12. Verfahren nach Anspruch 10 oder 11, bei dem wenigstens die mindestens eine Schutzeinrichtung (2) oder der Grundkörper (10) vor dem Beschichten aluminiert werden.

13. Verfahren nach Anspruch 12, wobei die mindestens eine Schutzeinrichtung (2) ein umgeformtes, insbesondere tiefgezogenes, Teil ist, bei dem der Aluminier-Vorgang vor dem Umformen stattfindet, und vorteilhafterweise nach dem Umformschritt ein Diffusionsglühen durchgeführt wird.

14. Verfahren nach einem der Ansprüche 10 bis 13, bei dem das Auftragen der Beschichtung (5) zumindest einen der folgenden Fertigungsschritte umfasst:
- Spritzen,
- Tauchen,
- Sprühen,
- Thermisches Spritzen.

15. Abgasreinigungskomponente umfassend zumindest eine Gassonde (1) gemäß einem der Ansprüche 1 bis 9.

16. Fahrzeug mit einem Abgassystem umfassen zumindest eine Gassonde (1) gemäß einem der Ansprüche 1 bis 9.

## Claims

1. A gas probe (1), in particular lambda probe for analyzing exhaust gases of a mobile internal combustion engine, having at least one protective device (2) which surrounds, at least in regions, a sensitive sensor element (3) of the gas probe (1), wherein the sensitive sensor element (3) comes into contact with a gas, **characterized in that** the at least one protective device (2) has a gas contact face (4) which at least partially has a hygroscopic surface whereby the hygroscopic surface is formed at least partially with a coating (5) which advantageously comprises a desiccant (6) which is resistant to high temperatures.

2. The gas probe (1) as claimed in claim 1, **characterized in that** the coating (5) has a layer thickness (7) in the range from 10 µm to 50 µm.

3. The gas probe (1) as claimed in claim 1 or 2, **characterized in that** the coating (5) has a porosity which is such that the pore volume per unit volume of the coating (5) is in a range from 30% to 90%.

4. The gas probe (1) as claimed in one of claims 1 to 3, **characterized in that** the coating (5) comprises an oxide, in particular at least one of the oxides titanium oxide, zirconium oxide and aluminum oxide.

5. The gas probe (1) as claimed in one of claims 1 to 4, **characterized in that** the coating (5) has constituents which have a catalytic action, in particular a noble metal (9).

6. The gas probe (1) as claimed in one of claims 1 to 5, **characterized in that** the further components which come into contact with the gas also at least partially have a hygroscopic surface, particularly a base body (10) of the gas probe (1).

7. The gas probe (1) as claimed in one of claims 1 to 6, **characterized in that** the at least one protective device (2) is a cap which comprises a material which is resistant to high temperatures and can be deep-drawn.

8. The gas probe (1) as claimed in one of claims 1 to 7, the gas probe (1) comprising at least the following components: at least one protective device (2) and a base body (10); **characterized in that** at least one component is made from a material which provides an oxide-forming surface.

9. The gas probe (1) according to one of claims 1 to8, **characterized in that** the at least one protective device (2) can be electrically heated.

10. A method for producing a gas probe (1) as claimed in one of the preceding claims, in particular a lambda probe for analyzing exhaust gases of a mobile internal combustion engine, having at least one protective device (2) which surrounds, at least in regions, a sensitive sensor element (3) of the gas probe (1), which sensitive sensor element (3) comes into contact with a gas, and having a base body (10), in which method at least the at least one protective device (2) is provided at least partially with a hygroscopic coating (5).

11. The method as claimed in claim 10, in which method the surfaces which are to be provided with the coating (5) are pre-treated, an oxide being formed in particular.

12. The method as claimed in 10 or 11, in which method at least the at least one protective device (2) or the base body (10) being aluminized before being coated.

13. The method as claimed in claim 12, the at least one protective device (2) being a shaped, in particular deep-drawn part, for which the aluminizing process takes place before the shaping process, and a homogenizing process is advantageously carried out after the shaping step.

14. The method as claimed in one of claims 10 to 13, in which method the application of the coating (5) comprises at least one of the following production steps:
- spraying,
- dipping,
- sputtering,
- thermal spraying.

15. An exhaust gas purification component comprising at least one gas probe (1) as claimed in one of claims 1 to 9.

16. A vehicle having an exhaust system comprising at least one gas probe (1) as claimed in one of claims 1 to 9.

## Revendications

1. Sonde de gaz (1), notamment sonde lambda pour analyser des gaz d'échappement d'une machine à combustion interne mobile, avec au moins un dispositif de protection (2), qui entoure au moins en parties un élément capteur (3) sensitif de la sonde de gaz (1), l'élément capteur (3) venant en contact avec un gaz, **caractérisée en ce que** l'au moins un dispositif de protection (2) a une surface de contact de gaz (4) qui a au moins partiellement une surface hygroscopique, la surface hygroscopique étant formée au moins partiellement avec un revêtement (5) qui comprend avantageusement un agent de déshydratation (6) résistant aux températures élevées.

2. Sonde de gaz (1) selon la revendication 1, **caractérisée en ce que** le revêtement (5) a une épaisseur de couche (7) dans la gamme de 10 µm à 50 µm.

3. Sonde de gaz (1) selon la revendication 1, **caractérisée en ce que** le revêtement (5) a une porosité, de sorte que le volume des pores par unité de volume du revêtement (5) se trouve dans une gamme de 30 % à 90 %.

4. Sonde de gaz (1) selon l'une des revendications 1 à 3, **caractérisée en ce que** le revêtement (5) comprend un oxyde, notamment au moins un parmi les composés de tytanyle, d'oxyde de zirconium et d'oxyde d'aluminium.

5. Sonde de gaz (1) selon l'une des revendications 1 à 4, **caractérisée en ce que** le revêtement est doté de constituants à activité catalytique, notamment d'un métal précieux (9).

6. Sonde de gaz (1) selon l'une des revendications 1 à 5, **caractérisée en ce que** les autres composants contactant également le gaz ont au moins partiellement une surface hygroscopique, notamment un corps de base (10) de la sonde de gaz (1).

7. Sonde de gaz (1) selon l'une des revendications 1 à 6, **caractérisée en ce que** l'au moins un dispositif de protection (2) est un capuchon qui comprend un matériau résistant aux températures élevées et pouvant être embouti.

8. Sonde de gaz (1) selon l'une des revendications 1 à 7, la sonde de gaz (1) comprenant au moins les composants suivants : au moins un dispositif de protection (2) et un corps de base (10) ; **caractérisée en ce qu'**au moins un composant est d'un matériau, qui met à disposition une surface à formation d'oxyde.

9. Sonde de gaz (1) selon l'une des revendications 1 à 8, **caractérisée en ce que** l'au moins un dispositif de protection (2) peut être chauffé électriquement.

10. Procédé destiné à la fabrication d'une sonde de gaz (1) selon l'une des revendications précédentes, notamment à une sonde lambda pour analyser des gaz d'échappement d'une machine à combustion interne mobile, avec au moins un dispositif de protection (2) qui entoure au moins en parties un élément capteur (3) sensitif de la sonde de gaz (1), l'élément capteur (3) contactant un gaz, ainsi qu'avec un corps de base (10) dans le cas duquel au moins l'au moins un dispositif de protection (2) est pourvu au moins partiellement d'un revêtement hygroscopique (5).

11. Dispositif selon la revendication 10, dans lequel les surfaces devant être pourvues du revêtement (5), sont prétraitées, un oxyde étant notamment formé.

12. Dispositif selon la revendication 10 ou 11, dans le cas duquel du moins l'au moins un dispositif de protection (2) ou le corps de base (10) sont aluminés avant d'être revêtus.

13. Dispositif selon la revendication 12, l'au moins un dispositif de protection (2) étant une pièce transformée, notamment emboutie, dans le cas de laquelle le processus d'aluminage a lieu avant la transformation et un recuit d'homogénéisation est effectué avantageusement après l'étape de transformation.

14. Procédé selon l'une des revendications 10 à 13, dans lequel l'application du revêtement (5) comprend au moins une des étapes de fabrication suivantes :
- l'injection,
- le trempage,
- l'aspergement,
- l'injection thermique.

15. Composant d'épuration de gaz d'échappement comprenant au moins une sonde de gaz (1) selon l'une des revendications 1 à 9.

16. Véhicule avec un système de gaz d'échappement, comprenant au moins une sonde de gaz (1) selon l'une des revendications 1 à 9.
